# EUROPEAN PATENT APPLICATION

(11) **EP 1 067 190 A1**
(43) Date of publication of application: **10.01.2001**
(21) Application number: 99202263.2
(22) Date of filing: 09.07.1999
(51) Int. Cl.: C12N 15/53, C12N 15/86, A61K 48/00, A61P 9/10

(54) **Gene therapy for enhancing and/or inducing angiogenesis**

(71) Applicant: Introgene B.V., 2333 AL Leiden (NL)
(72) Inventor: Vogels, Ronald, 3461 HW Linschoten (NL); Verlinden, Stefan, 2312 WB Leiden (NL)
(74) Representative: Ottevangers, Sietse Ulbe

(57) **Abstract**

The invention relates to gene therapy for enhancing and/or inducing angiogenesis, wherein use is made of a nucleic acid sequence encoding Nitric Oxide Synthase (NOS). In particular, the nucleic acid sequence is administered in a systemic treatment, preferably comprising isolated tissue perfusion.

### Annex to the application documents - subsequently filed sequences listing

SEQUENCE LISTING

<110> Introgene B.V.

<120> Gene therapy for enhancing and/or inducing
      angiogenesis.

<130> P49668EP00

<140> EP 99202263.2
<141> 1999-07-09

<160> 15

<170> PatentIn Ver. 2.1

<210> 1
<211> 23
<212> DNA
<213> Artificial Sequence

<220>
<223> Description of Artificial Sequence: oligo linker

<220>
<221> misc_feature
<222> (1)..(23)
<223> /note="Oligo linker containing a PacI site"

<400> 1
aattgtctta attaaccgct taa                                          23


<210> 2
<211> 19
<212> DNA
<213> Artificial Sequence

<220>
<223> Description of Artificial Sequence: oligonucleotide

<220>
<221> misc_feature
<222> (1)..(19)
<223> /note="Oligonucleotide"

<400> 2
aattgtctta attaaccgc                                               19


<210> 3
<211> 19
<212> DNA
<213> Artificial Sequence

<220>
<223> Description of Artificial Sequence: oligonucleotide

<220>
<221> misc_feature
<222> (1)..(19)

<223> /note="Oligonucleotide"

<400> 3
aattgcggtt aattaagac                                                    19


<210> 4
<211> 47
<212> DNA
<213> Artificial Sequence

<220>
<223> Description of Artificial Sequence: primer

<220>
<221> primer_bind
<222> (1)..(47)
<223> /note="Primer LTR-1"

<400> 4
ctgtacgtac cagtgcactg gcctaggcat ggaaaaatac ataactg                     47


<210> 5
<211> 64
<212> DNA
<213> Artificial Sequence

<220>
<223> Description of Artificial Sequence: primer

<220>
<221> primer_bind
<222> (1)..(64)
<223> /note="Primer LTR-2"

<400> 5
gcggatcctt cgaaccatgg taagcttggt accgctagcg ttaaccgggc gactcagtca 60
atcg                                                                    64


<210> 6
<211> 28
<212> DNA
<213> Artificial Sequence

<220>
<223> Description of Artificial Sequence: primer

<220>
<221> primer_bind
<222> (1)..(28)
<223> /note="Primer HSA1"

<400> 6
gcgccaccat gggcagagcg atggtggc                                          28


<210> 7
<211> 50
<212> DNA

<213> Artificial Sequence

<220>
<223> Description of Artificial Sequence: primer

<220>
<221> primer_bind
<222> (1)..(50)
<223> /note="Primer HSA2"

<400> 7
gttagatcta agcttgtcga catcgatcta ctaacagtag agatgtagaa          50


<210> 8
<211> 23
<212> DNA
<213> Artificial Sequence

<220>
<223> Description of Artificial Sequence: linker

<220>
<221> misc_feature
<222> (1)..(23)
<223> /note="PacI linker"

<400> 8
aattgtctta attaaccgct taa                                       23


<210> 9
<211> 67
<212> DNA
<213> Artificial Sequence

<220>
<223> Description of Artificial Sequence: oligonucleotide

<220>
<221> misc_feature
<222> (1)..(67)
<223> /note="Oligonucleotide PLL-1"

<400> 9
gccatcccta ggaagcttgg taccggtgaa ttcgctagcg ttaacggatc ctctagacga 60
gatctgg                                                         67


<210> 10
<211> 67
<212> DNA
<213> Artificial Sequence

<220>
<223> Description of Artificial Sequence: oligonucleotide

<220>
<221> misc_feature
<222> (1)..(67)
<223> /note="Oligonucleotide PLL-2"

<400> 10
ccagatctcg tctagaggat ccgttaacgc tagcgaattc accggtacca agcttcctag 60
ggatggc 67

<210> 11
<211> 39
<212> DNA
<213> Artificial Sequence

<220>
<223> Description of Artificial Sequence: primer

<220>
<221> primer_bind
<222> (1)..(39)
<223> /note="Primer CMVplus"

<400> 11
gatcggtacc actgcagtgg tcaatattgg ccattagcc 39

<210> 12
<211> 29
<212> DNA
<213> Artificial Sequence

<220>
<223> Description of Artificial Sequence: primer

<220>
<221> primer_bind
<222> (1)..(29)
<223> /note="Primer CMVminA"

<400> 12
gatcaagctt ccaatgcacc gttcccggc 29

<210> 13
<211> 1153
<212> PRT
<213> Homo sapiens

<220>
<221> CHAIN
<222> (1)..(1153)
<223> /note="Homo sapiens inducible nitric oxide synthase"

<400> 13

<210> 14
<211> 1433
<212> PRT
<213> Homo sapiens

<220>
<221> CHAIN
<222> (1)..(1433)
<223> /note="Human nitric oxide synthase"

<400> 14

<210> 15
<211> 1203
<212> PRT
<213> Homo sapiens

<220>
<221> CHAIN
<222> (1)..(1203)
<223> /note="Human nitric oxide synthase"

<400> 15
Met Gly Asn Leu Lys Ser Val Ala Gln Glu Pro Gly Pro Pro Cys Gly
  1               5                  10                  15

Leu Gly Leu Gly Leu Gly Leu Gly Leu Cys Gly Lys Gln Gly Pro Ala
             20                  25                  30

Thr Pro Ala Pro Glu Pro Ser Arg Ala Pro Ala Ser Leu Leu Pro Pro
         35                  40                  45

Ala Pro Glu His Ser Pro Pro Ser Ser Pro Leu Thr Gln Pro Pro Glu
     50                  55                  60

Gly Pro Lys Phe Pro Arg Val Lys Asn Trp Glu Val Gly Ser Ile Thr
 65                  70                  75                  80

Tyr Asp Thr Leu Ser Ala Gln Ala Gln Gln Asp Gly Pro Cys Thr Pro
                 85                  90                  95

Arg Arg Cys Leu Gly Ser Leu Val Phe Pro Arg Lys Leu Gln Gly Arg
            100                 105                 110

Pro Ser Pro Gly Pro Pro Ala Pro Glu Gln Leu Leu Ser Gln Ala Arg
        115                 120                 125

Asp Phe Ile Asn Gln Tyr Tyr Ser Ser Ile Lys Arg Ser Gly Ser Gln
    130                 135                 140

Ala His Glu Gln Arg Leu Gln Glu Val Glu Ala Glu Val Ala Ala Thr
145                 150                 155                 160

Gly Thr Tyr Gln Leu Arg Glu Ser Glu Leu Val Phe Gly Ala Lys Gln
                165                 170                 175

Ala Trp Arg Asn Ala Pro Arg Cys Val Gly Arg Ile Gln Trp Gly Lys
            180                 185                 190

Leu Gln Val Phe Asp Ala Arg Asp Cys Arg Ser Ala Gln Glu Met Phe
        195                 200                 205

Thr Tyr Ile Cys Asn His Ile Lys Tyr Ala Thr Asn Arg Gly Asn Leu
    210                 215                 220

Arg Ser Ala Ile Thr Val Phe Pro Gln Arg Cys Pro Gly Arg Gly Asp
225                 230                 235                 240

Phe Arg Ile Trp Asn Ser Gln Leu Val Arg Tyr Ala Gly Tyr Arg Gln
                245                 250                 255

Gln Asp Gly Ser Val Arg Gly Asp Pro Ala Asn Val Glu Ile Thr Glu
            260                 265                 270

Leu Cys Ile Gln His Gly Trp Thr Pro Gly Asn Gly Arg Phe Asp Val
        275                 280                 285

Leu Pro Leu Leu Leu Gln Ala Pro Asp Glu Pro Pro Glu Leu Phe Leu
    290                 295                 300

Leu Pro Pro Glu Leu Val Leu Glu Val Pro Leu Glu His Pro Thr Leu
305                 310                 315                 320

Glu Trp Phe Ala Ala Leu Gly Leu Arg Trp Tyr Ala Leu Pro Ala Val
                325                 330                 335

Ser Asn Met Leu Leu Glu Ile Gly Gly Leu Glu Phe Pro Ala Ala Pro
            340                 345                 350

Phe Ser Gly Trp Tyr Met Ser Thr Glu Ile Gly Thr Arg Asn Leu Cys
        355                 360                 365

Asp Pro His Arg Tyr Asn Ile Leu Glu Asp Val Ala Val Cys Met Asp
    370                 375                 380

Leu Asp Thr Arg Thr Thr Ser Ser Leu Trp Lys Asp Lys Ala Ala Val
385                 390                 395                 400

Glu Ile Asn Val Ala Val Leu His Ser Tyr Gln Leu Ala Lys Val Thr
                405                 410                 415

Ile Val Asp His His Ala Ala Thr Ala Ser Phe Met Lys His Leu Glu
            420                 425                 430

Asn Glu Gln Lys Ala Arg Gly Gly Cys Pro Ala Asp Trp Ala Trp Ile
        435                 440                 445

Val Pro Pro Ile Ser Gly Ser Leu Thr Pro Val Phe His Gln Glu Met
    450                 455                 460

Val Asn Tyr Phe Leu Ser Pro Ala Phe Arg Tyr Gln Pro Asp Pro Trp
465                 470                 475                 480

Lys Gly Ser Ala Ala Lys Gly Thr Gly Ile Thr Arg Lys Lys Thr Phe
                485                 490                 495

Lys Glu Val Ala Asn Ala Val Lys Ile Ser Ala Ser Leu Met Gly Thr
            500                 505                 510

Val Met Ala Lys Arg Val Lys Ala Thr Ile Leu Tyr Gly Ser Glu Thr
        515                 520                 525

Gly Arg Ala Gln Ser Tyr Ala Gln Gln Leu Gly Arg Leu Phe Arg Lys
    530                 535                 540

Ala Phe Asp Pro Arg Val Leu Cys Met Asp Glu Tyr Asp Val Val Ser
545                 550                 555                 560

Leu Glu His Glu Thr Leu Val Leu Val Val Thr Ser Thr Phe Gly Asn
                565                 570                 575

Gly Asp Pro Pro Glu Asn Gly Glu Ser Phe Ala Ala Ala Leu Met Glu
            580                 585                 590

Met Ser Gly Pro Tyr Asn Ser Ser Pro Arg Pro Glu Gln His Lys Ser
        595                 600                 605

Tyr Lys Ile Arg Phe Asn Ser Ile Ser Cys Ser Asp Pro Leu Val Ser
    610                 615                 620

Ser Trp Arg Arg Lys Arg Lys Glu Ser Ser Asn Thr Asp Ser Ala Gly
625                 630                 635                 640

Ala Leu Gly Thr Leu Arg Phe Cys Val Phe Gly Leu Gly Ser Arg Ala
                645                 650                 655

Tyr Pro His Phe Cys Ala Phe Ala Arg Ala Val Asp Thr Arg Leu Glu
            660                 665                 670

Glu Leu Gly Gly Glu Arg Leu Leu Gln Leu Gly Gln Gly Asp Glu Leu
        675                 680                 685

Cys Gly Gln Glu Glu Ala Phe Arg Gly Trp Ala Gln Ala Ala Phe Gln
    690                 695                 700

Ala Ala Cys Glu Thr Phe Cys Val Gly Glu Asp Ala Lys Ala Ala Ala
705                 710                 715                 720

Arg Asp Ile Phe Ser Pro Lys Arg Ser Trp Lys Arg Gln Arg Tyr Arg
                725                 730                 735

Leu Ser Ala Gln Ala Glu Gly Leu Gln Leu Leu Pro Gly Leu Ile His
            740                 745                 750

Val His Arg Arg Lys Met Phe Gln Ala Thr Ile Arg Ser Val Glu Asn
        755                 760                 765

Leu Gln Ser Ser Lys Ser Thr Arg Ala Thr Ile Leu Val Arg Leu Asp
    770                 775                 780

Thr Gly Gly Gln Glu Gly Leu Gln Tyr Gln Pro Gly Asp His Ile Gly
785                 790                 795                 800

Val Cys Pro Pro Asn Arg Pro Gly Leu Val Glu Ala Leu Leu Ser Arg
                805                 810                 815

Val Glu Asp Pro Pro Ala Pro Thr Glu Pro Val Ala Val Glu Gln Leu
            820                 825                 830

Glu Lys Gly Ser Pro Gly Gly Pro Pro Pro Gly Trp Val Arg Asp Pro
        835                 840                 845

Arg Leu Pro Pro Cys Thr Leu Arg Gln Ala Leu Thr Phe Phe Leu Asp
    850                 855                 860

Ile Thr Ser Pro Pro Ser Pro Gln Leu Leu Arg Leu Leu Ser Thr Leu
865                 870                 875                 880

Ala Glu Glu Pro Arg Glu Gln Gln Glu Leu Glu Ala Leu Ser Gln Asp

885                 890                 895

Pro Arg Arg Tyr Glu Glu Trp Lys Trp Phe Arg Cys Pro Thr Leu Leu
            900                 905                 910

Glu Val Leu Glu Gln Phe Pro Ser Val Ala Leu Pro Ala Pro Leu Leu
        915                 920                 925

Leu Thr Gln Leu Pro Leu Leu Gln Pro Arg Tyr Tyr Ser Val Ser Ser
    930                 935                 940

Ala Pro Ser Thr His Pro Gly Glu Ile His Leu Thr Val Ala Val Leu
945                 950                 955                 960

Ala Tyr Arg Thr Gln Asp Gly Leu Gly Pro Leu His Tyr Gly Val Cys
                965                 970                 975

Ser Thr Trp Leu Ser Gln Leu Lys Pro Gly Asp Pro Val Pro Cys Phe
            980                 985                 990

Ile Arg Gly Ala Pro Ser Phe Arg Leu Pro Pro Asp Pro Ser Leu Pro
        995                 1000                1005

Cys Ile Leu Val Gly Pro Gly Thr Gly Ile Ala Pro Phe Arg Gly Phe
    1010                1015                1020

Trp Gln Glu Arg Leu His Asp Ile Glu Ser Lys Gly Leu Gln Pro Thr
1025                1030                1035                1040

Pro Met Thr Leu Val Phe Gly Cys Arg Cys Ser Gln Leu Asp His Leu
                1045                1050                1055

Tyr Arg Asp Glu Val Gln Asn Ala Gln Gln Arg Gly Val Phe Gly Arg
            1060                1065                1070

Val Leu Thr Ala Phe Ser Arg Glu Pro Asp Asn Pro Lys Thr Tyr Val
        1075                1080                1085

Gln Asp Ile Leu Arg Thr Glu Leu Ala Ala Glu Val His Arg Val Leu
    1090                1095                1100

Cys Leu Glu Arg Gly His Met Phe Val Cys Gly Asp Val Thr Met Ala
1105                1110                1115                1120

Thr Asn Val Leu Gln Thr Val Gln Arg Ile Leu Ala Thr Glu Gly Asp
                1125                1130                1135

Met Glu Leu Asp Glu Ala Gly Asp Val Ile Gly Val Leu Arg Asp Gln
            1140                1145                1150

Gln Arg Tyr His Glu Asp Ile Phe Gly Leu Thr Leu Arg Thr Gln Glu
        1155                1160                1165

Val Thr Ser Arg Ile Arg Thr Gln Ser Phe Ser Leu Gln Glu Arg Gln
    1170                1175                1180

Leu Arg Gly Ala Val Pro Trp Ala Phe Asp Pro Pro Gly Ser Asp Thr
1185                1190                1195                1200

Asn Ser Pro

## Description

### FIELD OF THE INVENTION

The present invention relates to the field of gene therapy, more in particular to gene therapy vehicles and methods of delivery to stimulate the formation of new blood vessels (angiogenesis) in patients with endothelial dysfunction. For these purposes the invention discloses the delivery of therapeutic genes through the blood circulation.

### BACKGROUND OF THE INVENTION

Artherosclerosis is the accumulation of fatty deposits (plaque) inside blood vessels, leading to the blocking of the blood flow. Arteries throughout the body may be affected. The fibrous plaque forms occlusive lesions, because of its size and protusion into the arterial lumen. The fibrous cap covering the plague may rupture, leading to thrombus formation, resulting in further occlusion of the artery. When the lesion is located in the coronary artery, rupture will lead to a myocardial infarct. When the blood flow in brain vessels is blocked by lesions, this results in a stroke. In the limbs, the process of arterial narrowing will lead to ischemia, blocking of the vessel and finally to limb necrosis.

The early phases of atherosclerosis are characterised by endothelial dysfunction. Many therapies have been investigated to assess the possibility to reverse the endothelial dysfunction, and to stimulate the formation of new blood vessels (angiogenesis). It has recently been established that Nitric Oxide (NO) plays an important role in this process. Vascular endothelial dysfunction is characterised by the reduced release of NO in the arterial wall, which may cause a decrease in the blood flow in the arteries. Either process can lead to critical ischemia in the tissue drained by the affected vessel. Patients suffering from the consequences endothelial dysfunction could benefit from therapies to increase new collateral blood vessel formation.

It is known that angiogenesis is mediated by a multitude of cytokines (like TNF-α and E-selectin) and angiogenic factors including bFGF (basic Fibroblast Growth Factor), VEGF (Vascular Endothelial Growth Factor), and TGF-β. Both bFGF and VEGF are key regulators of angiogenesis in adult tissues. They selectively stimulate proliferation of endothelial cells, starting with the binding of these growth factors to receptors present on the endothelial cell surface. Nitric oxide (NO) has been shown to play a role in this process. NO, originally identified as endothelium-derived relaxing factor, is an important endothelial vasoactive factor.

While both NO and angiogenic factors like bFGF and VEGF play a key role in the endothelial functions, their precise mode of action is not known. On the one hand, levels of angiogenic factors like bFGF and VEGF are increased in patients suffering from endothelial dysfunction. On the other hand, the release of nitric oxide in dysfunctional vascular endothelium is often reduced. This reduced release may cause constriction of the coronary arteries and thus contribute to heart disease. It is postulated that patients suffering from endothelial dysfunction could benefit from therapies to increase new collateral blood vessel formation and/or therapies to increase vasodilation.

Many experimental gene therapies concentrate on the stimulation of angiogenesis, in patients suffering from endothelial dysfunction, through the addition of VGEF or bFGF. Though these experimental therapies may have some effect, the level of therapy induced angiogenesis is low, leading to a slow, if at all, recovery or enhancement of blood flow.

It has been demonstrated that NO is involved in VEGF-mediated proliferation of endothelial cells. Exposure of endothelial cells to VEGF was shown to lead to the activation of constitutive NO synthase (ceNOS, also called eNOS or NOSIII) and the release of biologically active NO. The proliferation of cells by VEGF can be inhibited by specific NOS-inhibitors like L-NAME, indicating that NO is an essential mediator in the VEGF-induced cell proliferation and angiogenesis.

Likewise, the presence of bFGF can increase ceNOS protein levels and enzyme activity during healing of rat gastric ulcers. Here also, the healing was inhibited specifically by the NOS-inhibitor L-NAME. In transgenic mouse models, disruption of the endogenous ceNOS gene impaired angiogenesis (Murohara et al.). This could not be compensated by the administration of VEGF, showing the essential role for NO in growth factor mediated angiogenesis.

The art teaches that a decreased NO synthesis in endothelial cells may limit new blood vessel formation in patients with endothelial dysfunction. It has been suggested that oral L-arginine supplementation in the diet may be a therapeutic strategy to improve angiogenesis in patients with endothelial dysfunction. Recent data in animal studies show that activation of the NO-pathway may actually lead to a regression of the pre-existing intimal lesions in atherosclerosis.

### SUMMARY OF THE INVENTION

It is an object of the present invention to provide a gene therapy for local administration of NO in blood vessels and surrounding tissues. It is also an object of the invention to induce angiogenesis in patients with endothelial dysfunction and to provide gene therapy methods for the treatment of atherosclerosis.

It has been found that vectors expressing at least a gene encoding Nitric Oxide Synthase (NOS), either alone or in combination with genes encoding angiogenic factors, can be used to transfect cells in ischemic areas to enhance angiogenesis in limbs, and thus restore blood flow. The synthesis of NO is regulated by a family of isozymes. Three isoforms are known: nNOS, ceNOS and iNOS. Both nNOS and ceNOS are constitutively expressed and tightly regulated by calmodulin, whereas iNOS is induced by the action of cytokines. In the context of the present invention, the term Nitric Oxide Synthase (NOS) is intended to encompass all members of the mentioned isozyme family.

In one aspect the invention provides a method for increasing NO and/or endothelial growth factors such as, but not limited to, VGEF and/or bFGF. In another aspect the invention provides a method for increasing vasodilation of blood vessels. In yet another aspect, the invention provides a method for increasing angiogenesis through locally delivering an expression vector, preferably an adenovirus vector, comprising at least a nucleic acid encoding NOS, to sites selected for being provided with the capacity to induce, or at least in part promote, angiogenesis.

Preferably, the delivery transpires through isolated tissue perfusion.

### BRIEF DESCRIPTION OF THE DRAWINGS

Figure 1: Schematic representation of the adapter plasmid pAd5/CLIP.

Figure 2: Schematic representation of the adapter plasmid Pad5/L420-HSApac.

Figure 3: Schematic representation of the adapter plasmid pAdApt.

Figure 4: Schematic representation of the adapter plasmid PAdApt-ceNOS.

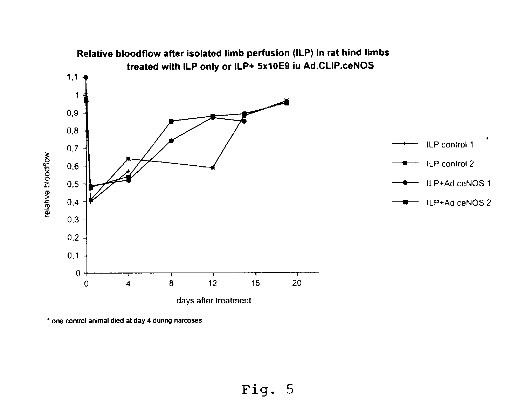

Figure 5: Relative bloodflow after isolated limb perfusion in rat hindlimb after occlusion of the artery and vena femoralis. Animals were treated with either isolated limb perfusion alone or isolated limb perfusion and delivery of 5x10⁹ Ad.CLIP.ceNOS infectious adenoviral particles. Blood flow was determined by Laser Doppler measurement of both footsoles. Using these measurements, the relative bloodflow was calculated by dividing the amount of bloodflow in the treated legs by the bloodflow of the untreated leg. Measurements were performed directly before and after the procedure and from thereon every 3-4 days until the relative bloodflow returned approximately to 1.

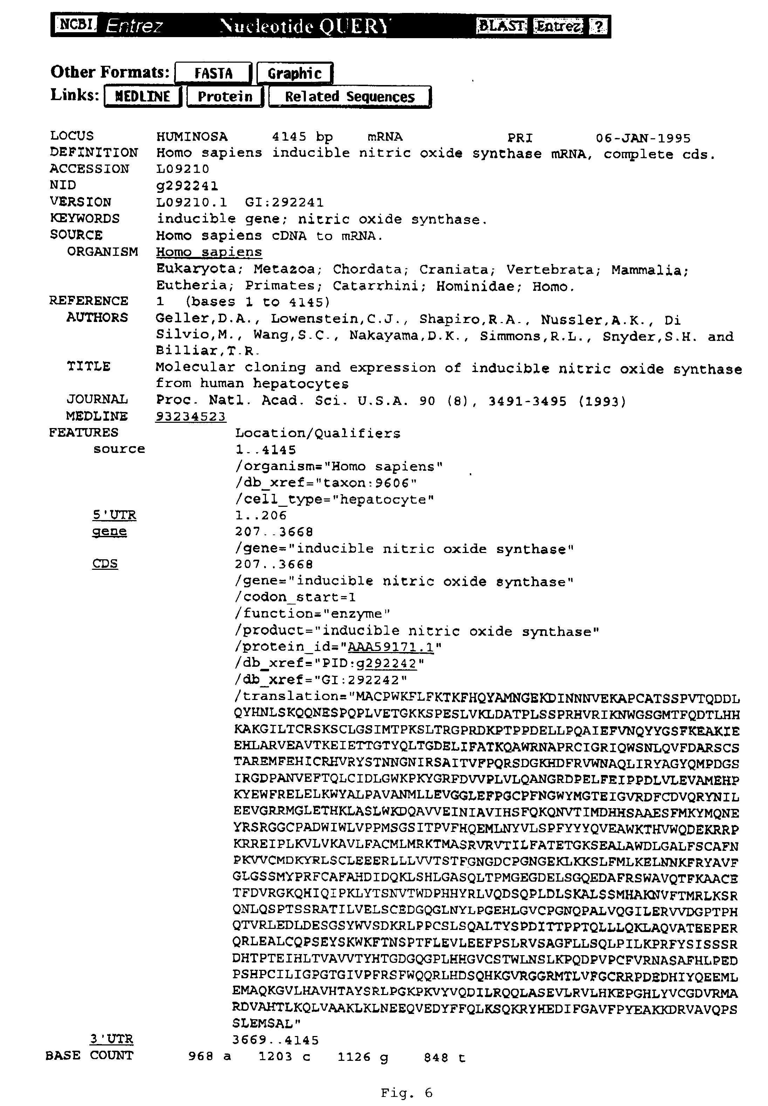

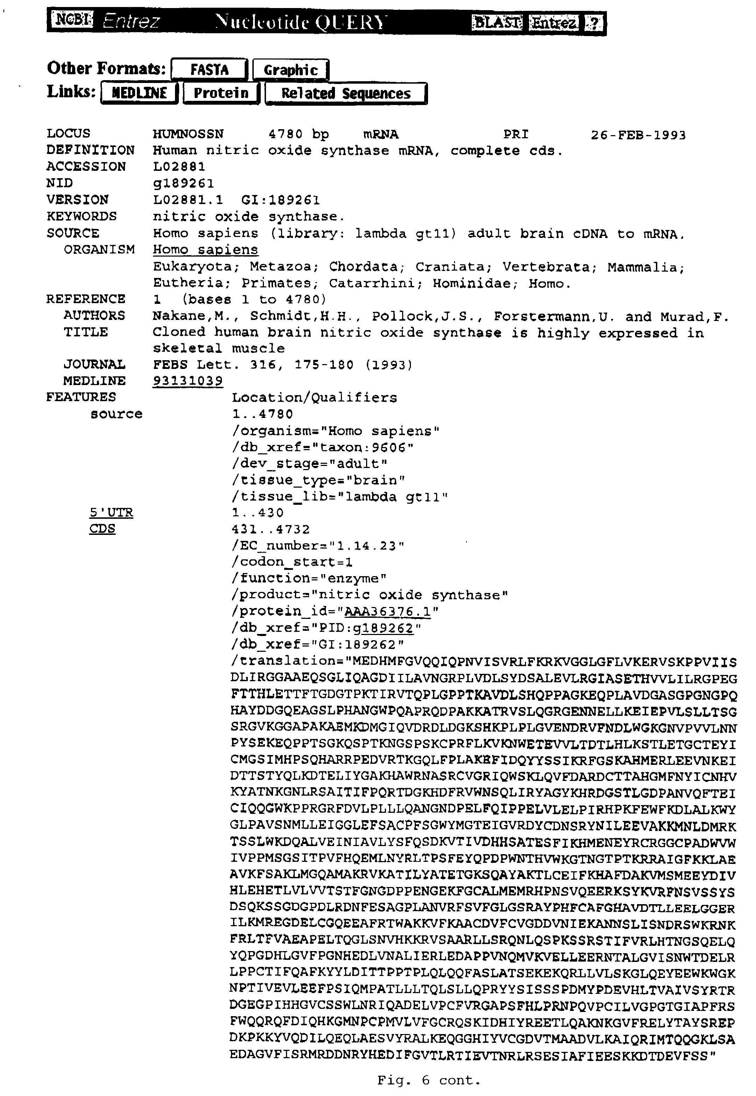

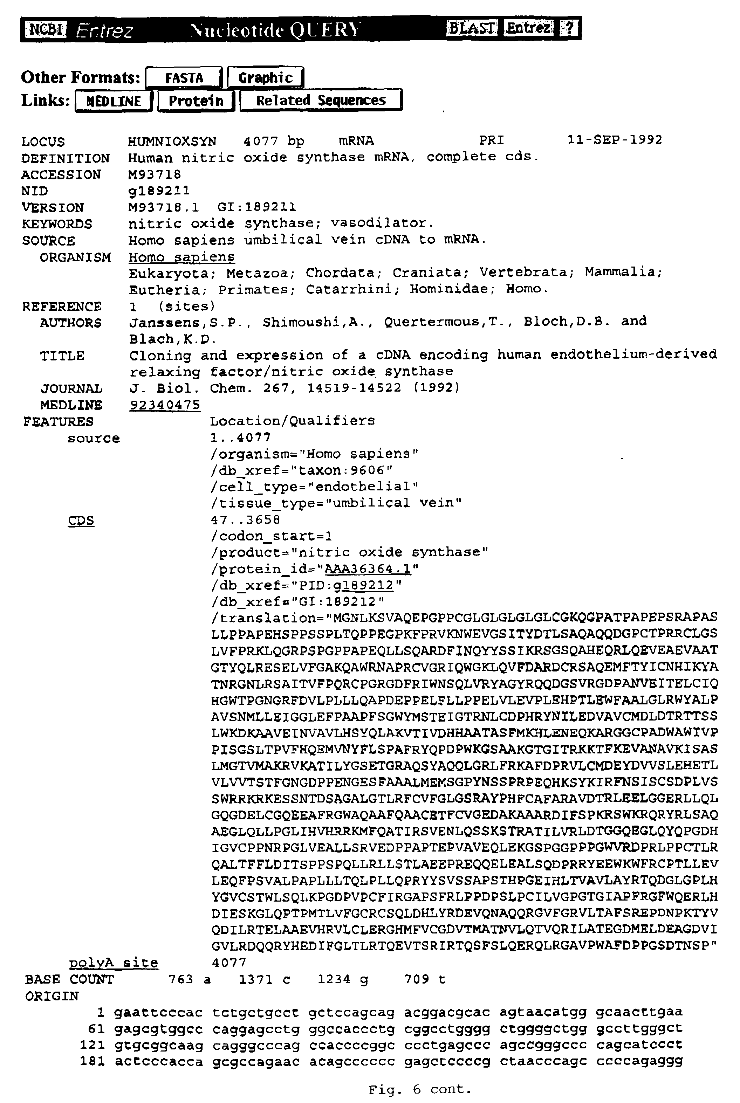

Figure 6. Nucleotide sequence of cloned NOS cDNAs

### DETAILED DESCRIPTION OF THE INVENTION

The present invention discloses the delivery of gene therapy vectors to a mammal carrying at least a NOS gene for use in enhancing vascularisation/blood flow in stenosed limbs after isolated limb perfusion (ILP). Because in most applications of the invention said mammal is a human, it is in most applications of the invention preferred that said nucleic acid molecule is a functional derivative from or includes at least a functional fragment of a nucleic acid molecule isolated from a human. The terms "functional derivative" and "functional fragment" are used here to indicate that said nucleic acid molecule encodes a peptide molecule with the same biological activity in kind, but not necessarily in amount, as said NOS.

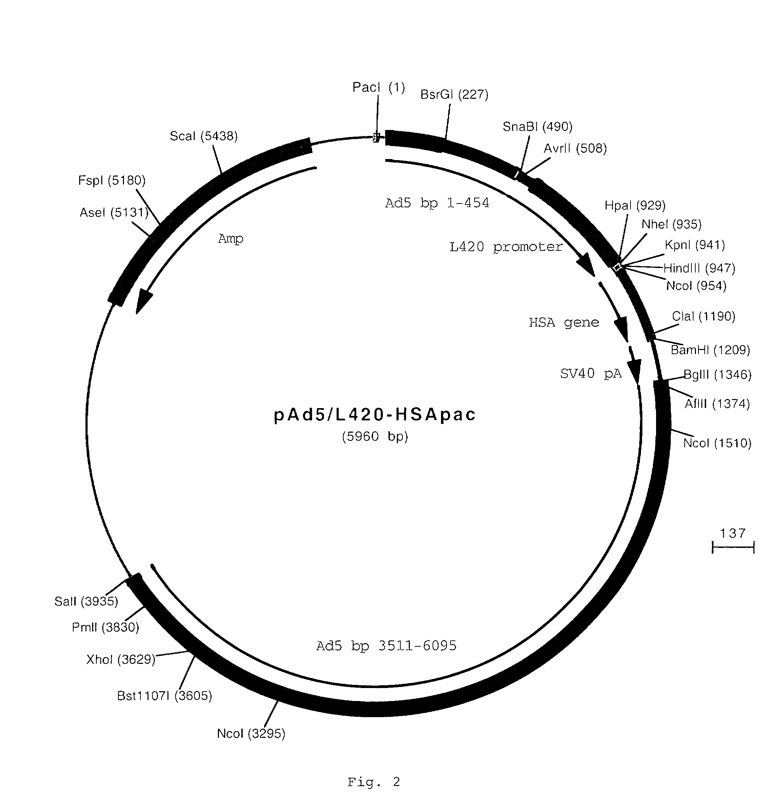

Three different isoforms of NOS have been identified. Two constitutively expressed isoforms are known, nNOS or NOSI, present in brain (Nakane *et al.,* 1993) and ceNOS or NOSIII, present in endothelial cells, (Janssens *et al.,* 1992). Both isofroms are dependent on calmodulin and Ca2+ for their activity. The third isoform (iNOS or NOSII, see Geller *et al.*, 1993) is Ca2+ independent, and its gene is induced by inflammation, microbial products and cytokines. Many cDNAs have been cloned and sequenced from different species and different tissues (see also figure 2). The isoforms share 50-60% sequence homology (For a recent review see Hobbs *et al., 1999).*

In gene therapy a molecule carrying genetic information is introduced in some or all cells of a host, whereby the genetic information is added to the host in a functional format.

For the purpose of gene therapy, nucleic acid delivery vehicles are commonly used to introduced foreign genetic information into target cells. Suitable nucleic acid delivery vehicles for the present invention are those nucleic acid delivery vehicles capable of delivering nucleic acid to cells *in vivo.* Non-limiting examples of such nucleic acid delivery vehicles are viral vectors, non-viral nucleic acid delivery vehicles and hybrids of viral and non-viral vehicles. Non-limiting examples of suitable viral vectors are adenovirus vectors, adeno-associated virus vectors and retroviral vectors. Non-limiting examples of non-viral nucleic acid delivery vehicles are liposomes, polyphosphazenes etc. In hybrid systems elements from viruses such as nucleic acid and/or proteins or parts thereof are incorporated into non-viral nucleic acid delivery vehicles to render the latter more effective.

Gene-transfer vectors derived from adenoviruses receive a lot of attention in the field of gene therapy. Adenoviruses are convenient viruses for construction of vectors for gene therapy, because of their high efficacy compared to other systems to deliver DNA in most mammalian cell types. Vectors derived from human adenoviruses, in which at least the E1 region has been deleted and replaced by a gene-of-interest, have been used extensively for gene therapy experiments in the pre-clinical and clinical phase.

There are several ways to administer recombinant adenovirus vectors. The recombinant virus can be injected intramuscularly, or be administered through a subcutaneous or a intravenous injection. These methods of administration have a disadvantage that leakage of the vector from the site of injection into the blood system leading to a diffuse uptake in other organs, especially the liver. Systemic delivery of adenovirus vectors has been hampered by the fact that this results mainly in uptake of the vectors by the liver [see: Connely S et al., "High level tissue specific expression of functional human factor VIII in mice", Human Gene Ther. 7(2):183-195 (1996); Herz J, Gerard, RD "Adenovirus mediated transfer of low density lipoprotein receptor gene acutely accelerates cholesterol clearance in normal mice", Proc. Natl. Acad. Sci. USA 90:2812-2816 (1993)]. Other organs are not or only minimally transduced by the adenovirus vectors.

The invention discloses the administration of a nucleic acid delivery vehicle without systemic delivery. Herein, an improved way of accessing ischemic areas in the limbs is provided, by delivering the vectors directly via the bloodstream. In accordance with the invention it is achieved that NO production in the endothelium is increased, thereby removing a cause for endothelial dysfunction. Preferably, said nucleic acid delivery vehicle comprises a virus-like particle. Preferably said virus-like particle is an adenovirus particle, an adeno-associated virus particle and/or a retrovirus particle.

In one embodiment of the invention, adenoviral vectors are employed to deliver these genes. In a further embodiment of the present invention, adenoviral vectors are provided that lack the early genes E1 and E2A. The recombinant adenoviral vectors according to the invention may be derived from any wild-type adenovirus serotype that allows the functional expression of said NOS in smooth muscle cells and/or in endothelial cells in the body of a mammal after administration of said recombinant adenoviral vector to the circulation of said mammal. Specifically when the induction angiogenesis is the aim of the treatment it is preferred to use a nucleic acid delivery vehicle capable of delivering said vehicle to preferably the smooth muscle cells lining the vessel wall. In this way angiogenesis promoting substances are delivered to a region localized close to the cells responsive for the substances or the products thereof. Furthermore, dilution of the substances and/or the products thereof into the blood is at least in part avoided, since the endothelial lining will at least in part prevent this. Striated muscle is not a tissue normally expressing NO and is therefore disfavoured as target cell for at least some of the NO-based angiogenesis promotion applications.

The present invention is exemplified on the based of adenovirus vectors but is not limited to adenovirus vector. In the examples given infra to illustrate the present invention, said recombinant adenoviral vectors are derived from human adenovirus type 5. Typically, one would like to optimize the delivery of the nucleic acid defined supra, particularly to cells of the vessel wall, particularly endothelial cells and/or smooth muscle cells. For this, reason in one embodiment of the invention the nucleic acid delivery vehicle comprises a fiber protein derived from an adenovirus of a different subgroup than subgroup C, the subgroup that adenovirus serotype 5 belongs to. Preferably, said different subgroup is subgroup B, although subgroup D and/or F are also suitable. Preferably, said adenovirus of subgroup B is adenovirus 16 or adenovirus 35. It is to be understood, however, that those skilled in the art will be able to apply other viral vectors, such as other recombinant adenoviral vectors, without departing from the invention. Methods for the construction of recombinant adenoviral vectors according to the invention and for their propagation on useful packaging cells have been described in patent applications EP 0 707 071 and WO 97/00326, incorporated herein by reference. Other examples of vectors and packaging systems useful in the invention include, but are not limited to, those given in patent applications WO 93/19191, WO 94/28152, WO 96/10642, and WO 97/04119.

The invention provides means and methods for isolated tissue perfusion, preferably isolated limb perfusion (ILP), in which the blood circulation of the limb is isolated from the circulation of the body. The circulation through the limb is maintained by a pump. We have perfused the limb with an E1 deleted recombinant adenovirus vector harbouring a human ceNOS gene. Thus in a preferred embodiment the invention provides a systemic treatment which includes isolated tissue perfusion.

Tissue perfusion is intended to read on isolated tissues as well as organs and/or extremities or any combination thereof. Two approaches of isolated perfusion are provided, one in which cells in the isolated perfunded tissue are target cells for the delivery of nucleic acid and one in which the target cells for the delivery of nucleic acid are not in the isolated perfunded tissue. Organs or body parts which are liable to be damaged by the treatment or which are likely to influence the uptake of virus by the target cells or which need to be isolated for a different reason can be excluded from the system to which the adenoviral vector encoding NOS activity is provided. For instance, liver delivery is for some applications not preferred. For instance with the current thymidine kinase suicide approaches it is better to avoid any possibility of toxicity for liver cells, since there are indications in mice that this organ is more sensitive for the cell killing effects of the treatment than other tissues. In the other isolated perfusion route the vector is delivered to the isolated part only. In one embodiment delivery of nucleic acid through isolated tissue perfusion of the heart is provided. In another embodiment, delivery of nucleic acid through isolated tissue perfusion of the liver is provided. Said organs may be isolated for the normal circulation and perfunded through methods known in the art and can be combined with the means and methods of the invention to improve delivery of the nucleic acid of interest. It is preferred to deliver the vector in the form of a virus-like particle. This means that the vector is packed in an virus shell, preferably an adenovirus shell, an adeno-associated virus shell or a retrovirus shell.

The present invention furthermore provides a pharmaceutical composition that comprises the nucleic acid delivery vehicle defined *supra* in combination with a diluent that is not toxic to the recipient mammal at the dosage used and that retains sufficient stability of the infectivity of said nucleic acid delivery vehicle for a time long enough to allow uptake of said nucleic acid delivery vehicle into the muscle cells and/or endothelial cells after administration of said composition to the circulation of the recipient mammal. Preferably, said nucleic acid delivery vehicle comprises an adenovirus vector. A typical non-limiting example of a diluent according to this aspect of the invention is an isotonic saline solution that is sterile and that is buffered at a physiological pH. Preferably, said diluent furthermore contains serum-substituting ingredients. In the examples given infra to illustrate the present invention Haemaccel (Behring Pharma) is used as a suitable diluent. It is to be understood, however, that those skilled in the art will be able to apply other diluents without departing from the invention. For some applications of the invention it is furthermore preferred that said pharmaceutical composition is oxygenated prior to administration. Optionally, said nucleic acid delivery vehicle (the recombinant adenoviral vector and/or virus) is prepared in lyophilized form. In the latter case, said nucleic acid delivery vehicle is suspended in solution to obtain said pharmaceutical composition before administering said pharmaceutical composition to the circulation of the recipient mammal. Typically, a pharmaceutical composition comprising one dose of the virus-like particle defined supra, contains at least about 10⁶, preferably about 10⁸ infectious units (iu) of the virus-like particle of the invention, but in certain conditions it is preferred that it contains at least about 10⁹, more preferred 10¹⁰, or even more preferred 10¹¹ iu. The amount of virus to be provided depends on many parameters. As disclosed herein only a very limited portion of the administered virus actually infects the target cells. This may be one reason to increase the amount of virus to be administered. Another important aspect is of course the amount of NOS activity expressed by a cell infected with one or more viruses. This of course depends on the cell, but also on the promoter that drives the expression and its interaction with cell components of the expression machinery, etc.

In another aspect, the invention provides a method to deliver said nucleic acid molecule that encodes NOS to smooth muscle cells and/or endothelial cells in the body of a mammal, whereby the adenoviral vector or pharmaceutical composition defined supra is administered to a site in the circulation of said mammal. Said circulation is meant to include both the blood circulation and the lymphatic circulation. Thus, the administration is performed to any site in the body of the recipient mammal where the blood or lymph fluids of said mammal pass. To more accurately restore blood flow, administrations of said nucleic acid delivery vehicle are preferably performed in conducting-arteria (intra-arterial or intravenous), where it is further preferred that said administration is into an artery located upstream of the ischemic area. Preferably, rather than expanding the capillary vessel network, novel conducting vessels are generated. Typically occlusion occur in the conducting vessels, therefore typically expanding the capillary vessel network will have at best a limited effect on blood flow. The delivery of said nucleic acid delivery vehicle to conducting vessels will at least in part allow the preferred generation of novel conduction vessels. There are several means to perform said administration to the circulation. One of said means is by injection using, e.g., a syringe, a catheter or another infusion system known in the art. Preferably, said injection is performed at a controlled infusion rate. A much preferred means to perform said administration to the circulation is by perfusion. Perfusion is a technique whereby said administered pharmaceutical composition is caused to pass through said circulation or through a part of said circulation. When the administration is performed by perfusion it is furthermore preferred that said perfusion is done multiple times by creating a closed circuit and repassaging said pharmaceutical composition through said circulation or said part of circulation. Typically, said causing to pass is done by using a pump device and perfusion is performed at a rate depending on the species of the mammal to which said pharmaceutical composition is being administered. For humans, said rate is often in the range of approximately 40-80 ml/min and said perfusion is continued for a period of 15-90 minutes, but depending on patient, type of vascular endothelial dysfunction, and location thereof. These parameters may vary. For short treatment times (approximately 5-30 minutes) with the adenoviral construct an anoxic perfusion can be performed by those skilled in the art by using balloon catheters to make a closed circuit. No heart-lung machine is necessary.

For optimal delivery of said nucleic acid molecule that encodes a Nitric Oxide Synthase, to the target cells, preferably, smooth muscle cells and/or endothelial cells, it is furthermore preferred that the blood of the mammal is first essentially washed away from said closed circuit (e.g., by precirculation with the diluent of the pharmaceutical composition only) before said pharmaceutical composition is administered. Optionally, the blood that is washed away is collected and readministered at the end of the procedure. The perfusion liquid can be oxygenated if needed. Essentially washing the blood from the closed circuit, allows at least in part removal of antibodies that may affect the transduction procedure. Such may be the cases when the blood contains or is suspected of containing, neutralizing antibodies against the nucleic acid delivery vehicle. Surgical techniques for perfusion of parts of the circulation according to the present invention are under development and are already available for various specific parts of the circulation, such as, e.g., the liver (Fraker, DL et al., Circulatory shock, 44, p.45-50, 1994), the lung (Progrebniak HW et al., Ann. Thorac. Surg.,57, p.1477-83, 1994), and the kidney (Veen van de AH et al., Eur. J. Surg. Oncol. 20, p.404-405, 1994). A typical non-limiting example of a routine perfusion technique useful in the invention is isolated limb perfusion (ILP), where a closed circuit is created between the femoral artery and the femoral vein. Alternatively, essentially the same perfusion techniques can be employed in the invention to exclude the delivery of said nucleic acid molecule to a part or parts of the circulation. In this aspect of the invention, the part or parts of the circulation to which said delivery is unwanted are perfused with a diluent according to the invention while said pharmaceutical composition is administered to the circulation systemically (hence, outside the perfusion circulation). An important example of this embodiment of the invention is exclusion of the liver circulation from delivery of said nucleic acid molecule.

The invention will now be elucidated by the following, non-restrictive examples.

### EXAMPLES

### Example 1

### Plasmid-based system for rapid RCA-free generation of recombinant adenoviral vectors

### A. Construction of adenovirus clones pBr/Ad.Bam-rITR (ECACC deposit P970821212

In order to facilitate blunt end cloning of the ITR sequences, wild-type human adenovirus type 5 (Ad5) DNA was treated with Klenow enzyme in the presence of excess dNTPs. After inactivation of the Klenow enzyme and purification by phenol/chloroform extraction followed by ethanol precipitation, the DNA was digested with *Bam*HI. This DNA preparation was used without further purification in a ligation reaction with pBr322 derived vector DNA prepared as follows: pBr322 DNA was digested with EcoRV and *Bam*HI, dephosphorylated by treatment with TSAP enzyme (Life Technologies) and purified on LNP agarose gel (SeaPlaque GTG). After transformation into competent *E. coli* DH5α (Life Techn.) and analysis of ampicillin resistant colonies, one clone was selected that showed a digestion pattern as expected for an insert extending from the *Bam*HI site in Ad5 to the right ITR. Sequence analysis of the cloning border at the right ITR revealed that the most 3' G residue of the ITR was missing, the remainder of the ITR was found to be correct. Said missing G residue is complemented by the other ITR during replication.

### pBr/Ad.Cla-Bam (ECACC deposit P97082117)

wt Adeno type 5 DNA was digested with *Cla*I and *Bam*HI, and the 20.6 kb fragment was isolated from gel by electro-elution. pBr322 was digested with the same enzymes and purified from agarose gel by Geneclean. Both fragments were ligated and transformed into competent DH5α. The resulting clone pBr/Ad.Cla-Bam was analyzed by restriction enzyme digestion and shown to contain an insert with adenovirus sequences from bp 919 to 21566.

### pBr/Ad.AfII-Bam (ECACC deposit P97082114)

Clone pBr/Ad.Cla-Bam was linearized with *Eco*RI (in pBr322) and partially digested with *Afl*II. After heat inactivation of *Afl*II for 20 minutes at 65°C, the fragment ends were filled in with Klenow enzyme. The DNA was then ligated to a blunt double stranded oligo linker containing a PacI site (5'-AATTGTCTTAATTAACCGCTTAA-3'). This linker was made by annealing the following two oligonucleotides: 5'-AATTGTCTTAATTAACCGC-3' and 5'-AATTGCGGTTAATTAAGAC-3', followed by blunting with Klenow enzyme. After precipitation of the ligated DNA to change buffer, the ligations were digested with an excess PacI enzyme to remove concatameres of the oligo. The 22016 bp partial fragment containing Ad5 sequences from bp 3534 up to 21566 and the vector sequences, was isolated in LMP agarose (SeaPlaque GTG), religated and transformed into competent DH5a. One clone that was found to contain the PacI site and that had retained the large adeno fragment was selected and sequenced at the 5' end to verify correct insertion of the *PacI* linker in the (lost) *Afl*II site.

### pBr/Ad.Bam-rITRpac#2 (ECACC deposit P97082120) and pBr/Ad.Bam-rITR#8 (ECACC deposit P97082121)

To allow insertion of a *Pac*I site near the ITR of Ad5 in clone pBr/Ad.Bam-rITR about 190 nucleotides were removed between the *Cla*I site in the pBr322 backbone and the start of the ITR sequences. This was done as follows: pBr/Ad.Bam-rITR was digested with *Cla*I and treated with nuclease Bal31 for varying lengths of time (2', 5', 10' and 15'). The extent of nucleotide removal was followed by separate reactions on pBr322 DNA (also digested at the *Cla*I site), using identical buffers and conditions. *Bal*31 enzyme was inactivated by incubation at 75°C for 10 minutes, the DNA was precipitated and resuspended in a smaller volume TE buffer. To ensure blunt ends, DNAs were further treated with T4 DNA polymerase in the presence of excess dNTPs. After digestion of the (control) pBr322 DNA with *Sal*I, satisfactory degradation (^{~}150 bp) was observed in the samples treated for 10 minutes or 15 minutes. The 10 minutes or 15 minutes treated pBr/Ad.Bam-rITR samples were then ligated to the above described blunted *PacI* linkers (see pBr/Ad.*Afl*II-Bam). Ligations were purified by precipitation, digested with excess *PacI* and separated from the linkers on an LMP agarose gel. After religation, DNAs were transformed into competent DH5a and colonies analyzed. Ten clones were selected that showed a deletion of approximately the desired length and these were further analyzed by T-track sequencing (T7 sequencing kit, Pharmacia Biotech). Two clones were found with the *Pacl* linker inserted just downstream of the RITR. After digestion with *Pacl,* clone #2 has 28 bp and clone #8 has 27 bp attached to the ITR.

### pWE/Ad.AflII-rITR (ECACC deposit P97082116)

Cosmid vector pWE15 (Clontech) was used to clone larger Ad5 inserts. First, a linker containing a unique *PacI* site was inserted in the *Eco*RI sites of pWE15 creating pWE.pac. To this end, the double stranded *Pac*I oligo as described for pBr/Ad.AflII-BamHI was used but now with its *Eco*RI protruding ends. The following fragments were then isolated by electro-elution from agarose gel:pWE.pac digested with *Pac*I, pBr/AflII-Bam digested with *Pac*I and *Bam*HI and pBr/Ad.Bam-rITR#2 digested with *Bam*HI and *Pac*I. These fragments were listed together and packaged using λ phage packaging extracts (Stratagene) according to the manufacturer's protocol. After infection into host bacteria, colonies were grown on plates and analyzed for presence of the complete insert. pWE/Ad.AfIII-rITR contains all adenovirus type 5 sequences, from bp 3534 (*Afl*II site) up to and including the right ITR (missing the most 3' G residue).

### B. Construction of new adapter plasmids

### Generation of adapter plasmid pAd/L420-HSApac

The absence of sequence overlap between the recombinant adenovirus and E1 sequences in the packaging cell line is essential for safe, RCA-free generation and propagation of new recombinant viruses. The adapter plasmid pMLPI.TK (described in WO 97/00326) is an example of an adapter plasmid designed for use in combination with improved packaging cell lines like PER.C6 (described in WO 97/00326 and US 08/892,873). This plasmid was used as the starting material to make new adapter plasmids in which nucleic acid molecules comprising specific promoter and gene sequences can be easily exchanged.
First, a PCR fragment was generated from pZipΔMo+PyF101(N⁻) template DNA (described in PCT/NL96/00195) with the following primers: LTR-1: 5'-CTG TAC GTA CCA GTG CAC TGG CCT AGG CAT GGA AAA ATA CAT AAC TG-3' and LTR-2: 5'-GCG GAT CCT TCG AAC CAT GGT AAG CTT GGT ACC GCT AGC GTT AAC CGG GCG ACT CAG TCA ATC G-3'. Pwo DNA polymerase (Boehringer Mannheim) was used according to manufacturers protocol with the following temperature cycles: once 5' at 95°C; 3' at 55°C; and 1' at 72°C, and 30 cycles of 1' at 95°C, 1' at 60°C, 1' at 72°C, followed by once 10' at 72°C. The PCR product was then digested with BamHI and ligated into pMLP10 (Levrero et al., 1991) vector digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Next, the coding region of the murine HSA gene was inserted. pLTR10 was digested with BstBI followed by Klenow treatment and digestion with NcoI. The HSA gene was obtained by PCR amplification on pUC18-HSA (Kay et *al.,* 1990) using the following primers: HSA1, 5'-GCG CCA CCA TGG GCA GAG CGA TGG TGG C-3' and HSA2, 5'-GTT AGA TCT AAG CTT GTC GAC ATC GAT CTA CTA ACA GTA GAG ATG TAG AA-3'. The 269 bp amplified fragment was subcloned in a shuttle vector using the NcoI and BglII sites. Sequencing confirmed incorporation of the correct coding sequence of the HSA gene, but with an extra TAG insertion directly following the TAG stop codon.The coding region of the HSA gene, including the TAG duplication was then excised as a NcoI(sticky)-SalI(blunt) fragment and cloned into the 3.5 kb NcoI(sticky)/BstBI(blunt) fragment from pLTR10, resulting in pLTR-HSA10.
Finally, pLTR-HSA10 was digested with EcoRI and BamHI after which the fragment containing the left ITR, packaging signal, L420 promoter and HSA gene was inserted into vector pMLPI.TK digested with the same enzymes and thereby replacing the promoter and gene sequences. This resulted in the new adapter plasmid pAd/L420-HSA that contains convenient recognition sites for various restriction enzymes around the promoter and gene sequences.

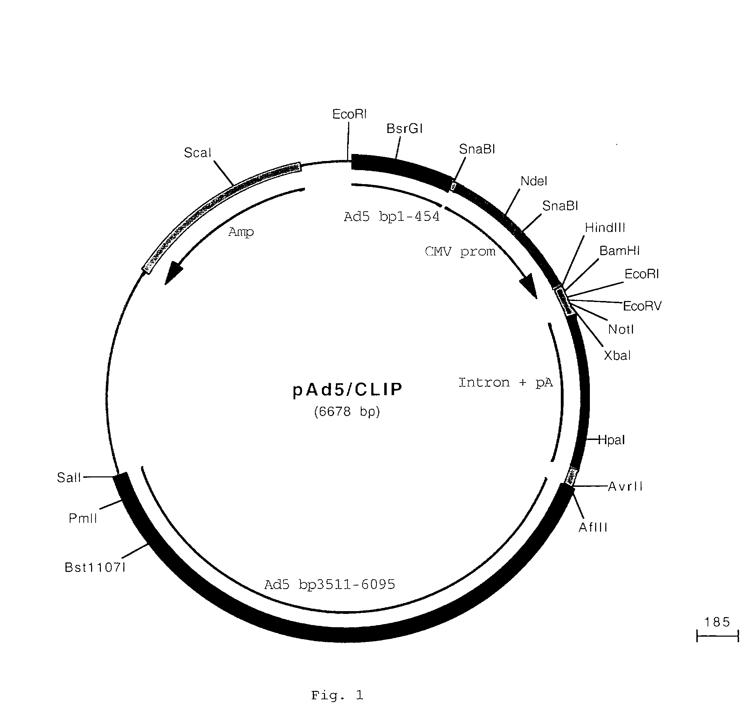

Another adapter plasmid that was designed to allow easy exchange of nucleic acid molecules was made by replacing the promoter, gene and poly A sequences in pAd/L420-HSA with the CMV promoter, a multiple cloning site, an intron and a poly-A signal. For this purpose, pAd/L420-HSA was digested with AvrII and BglII followed by treatment with Klenow to obtain blunt ends. The 5.1 kb fragment with pBr322 vector and adenoviral sequences was isolated and ligated to a blunt 1570 bp fragment from pcDNAl/amp (Invitrogen) obtained by digestion with HhaI and AvrII followed by treatment with T4 DNA polymerase. This adapter plasmid was named pAd5/CLIP (Figure 1). To enable removal of vector sequences from the left ITR in pAd5/Clip, this plasmid was partially digested with EcoRI and the linear fragment was isolated. An oligo of the sequence 5' TTAAGTCGAC-3' was annealed to itself resulting in a linker with a SalI site and EcoRI overhang. The linker was ligated to the partially digested pAd5/Clip vector and clones were selected that had the linker inserted in the EcoRI site 23 bp upstream of the left adenovirus ITR in pAd5/Clip resulting in pAd5/Clipsal.
The vector pAd5/L420-HSA was then modified to create a SalI or PacI site upstream of the left ITR. Hereto pAd5/L420-HSA was digested with EcoRI and ligated to a PacI linker (5'-AATTGTCTTAATTAACCGCTTAA-3'). The ligation mixture was digested with PacI and religated after isolation.of the linear DNA from agarose gel to remove concatamerised linkers. This resulted in adapter plasmid pAd5/L420-HSApac (Figure 2).

### Generation of adapter plasmids pAdMire and pAdApt

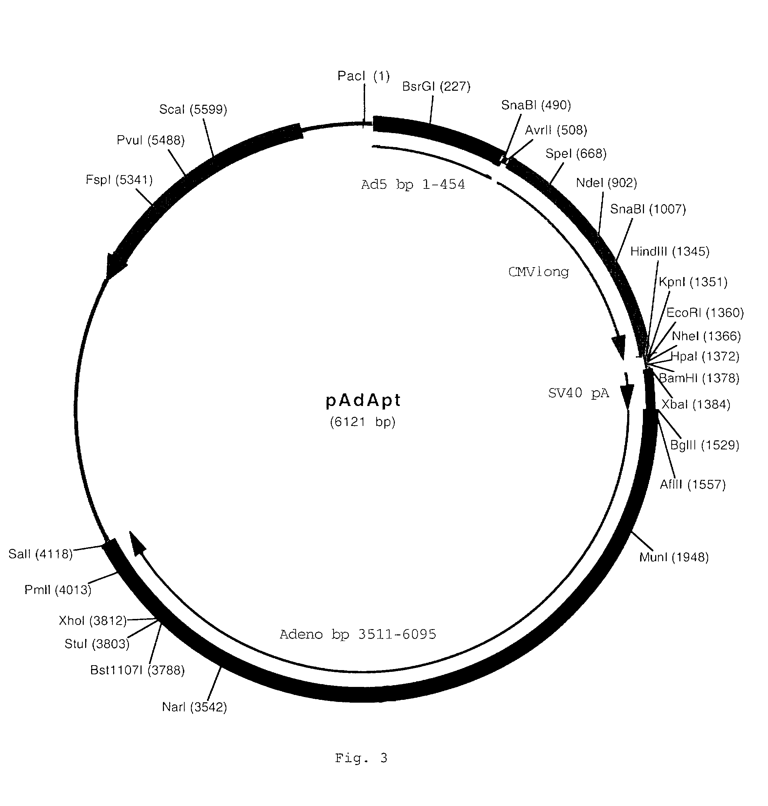

To create an adapter plasmid that only contains a polylinker sequence and no promoter or polyA sequences, pAd5/L420-HSApac was digested with AvrII and BglII. The vector fragment was ligated to a linker oligonucleotide digested with the same restriction enzymes. The linker was made by annealing oligos of the following sequence: 
PLL-1: 5'- GCC ATC CCT AGG AAG CTT GGT ACC GGT GAA TTC GCT
AGC GTT AAC GGA TCC TCT AGA CGA GAT CTG G-3'
 and 
PLL-2: 5'- CCA GAT CTC GTC TAG AGG ATC CGT TAA CGC TAG CGA
ATT CAC CGG TAC CAA GCT TCC TAG GGA TGG C-3'.
 The annealed linkers were digested with AvrII and BglII and separated from small ends by column purification (Qiaquick nucleotide removal kit) according to manufacterers recommendations. The linker was then ligated to the AvrII/BglII digested pAd5/L420-HSApac fragment. A clone, named pAdMire, was selected that had the linker incorporated and was sequenced to check the integrity of the insert. Adapter plasmid pAdMire enables easy insertion of complete expression cassettes.
An adapter plasmid containing the human CMV promoter that mediates high expression levels in human cells was constructed as follows: pAd5/L420-HSApac was digested with AvrII and 5' protruding ends were filled in using Klenow enzyme. A second digestion with HindIII resulted in removal of the L420 promoter sequences. The vector fragment was isolated and ligated to a PCR fragment containing the CMV promoter sequence. This PCR fragment was obtained after amplification of CMV sequences from pCMVLacI (Stratagene) with the following primers: CMVplus: 5'-GATCGGTACCACTGCAGTGGTCAATATTGGCCATTAGCC-3' and CMVminA: 5'-GATCAAGCTTCCAATGCACCGTTCCCGGC-3'. The PCR fragment was first digested with PstI (underlined in CMVplus) after which the 3'-protruding ends were removed by treatment with T4 DNA polymerase. Then the DNA was digested with HindIII (underlined in CMVminA) and ligated into the above described pAd5/L420-HSApac vector fragment digested with AvrII and HindIII. The resulting plasmid was named pAd5/CMV-HSApac. This plasmid was then digested with HindIII and BamHI and the vector fragment was isolated and ligated to the polylinker sequence obtained after digestion of pAdMire with HindIII and BglII. The resulting plasmid was named pAdApt (Figure 3). Adapter plasmid pAdApt contains nucleotides -735 to +95 of the human CMV promoter (Boshart et al., 1985; A very strong enhancer is located upstream of an immediate early gene of human cytomegalovirus. Cell 41,521-530, 1985).

### Generation of pAdApt-ceNOS

Plasmid pAC(d)CMVceNOS (described in Janssens *et al.* 1998; Human endothelial nitric oxide synthase gene transfer inhibits vascular smooth muscle cell proliferation and neointima formation after balloon injury in rats. Circulation 97, 1274-1281) was digested with EcoRI and the ends were filled in using Klenow enzyme. The ceNOS insert was then removed by digestion with XbaI and isolated from gel using the GeneClean kit II (Bio 101 Inc.). pAd/Clip was digested with BamHI and the ends were also filled in using Klenow followed by digestion with XbaI and isolation from gel. Ligation of the two fragments resulted in pAd/Clip-ceNOS.

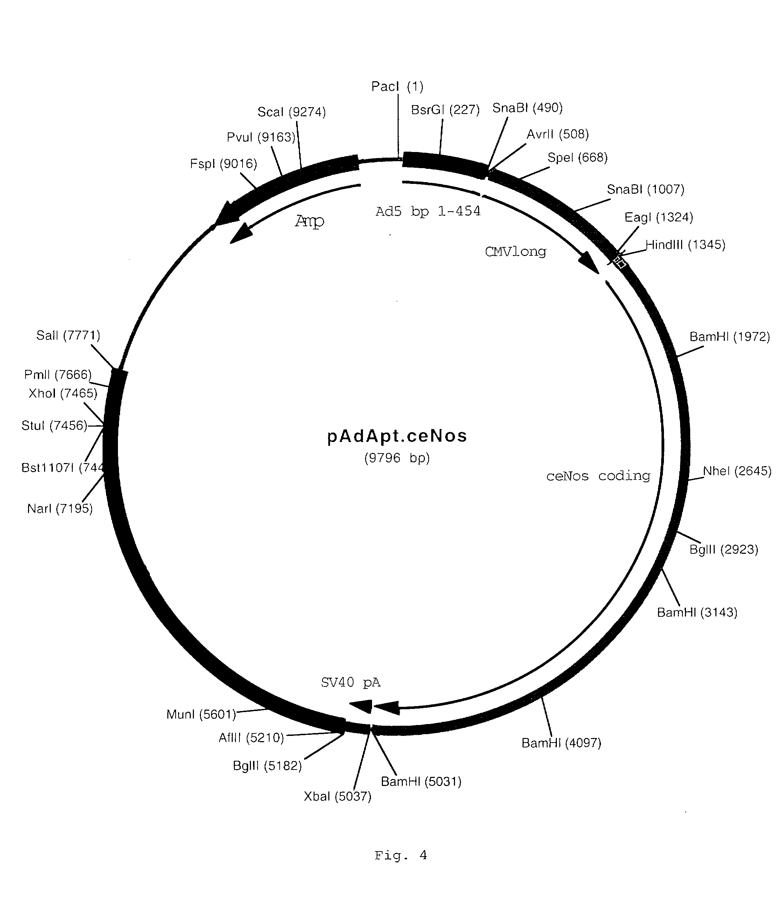

The ceNOS sequence was removed from pAd5/Clip-ceNOS by digestion with HindIII and XbaI and the 3.7 kb ceNOS fragment was isolated from gel using the GeneClean spinkit (Bio101 Inc.) according to the manufacterers instructions. Adapter plasmid pAdApt was also digested with HindIII and XbaI and the linear fragment was isolated as described above. Both fragments were ligated resulting in pAdApt-ceNOS (Figure 4).

The recombinant adenoviruses IGAdApt and IGAdApt-ceNOS were generated using the above described adapter plasmids and the adenovirus cosmid clone pWE/Ad.AflII-rITR.

### C. Generation of recombinant adenoviruses

### El-deleted recombinant adenoviruses

To generate E1 deleted recombinant adenoviruses with the new plasmid-based system, the following constructs were prepared: an adapter construct containing the expression cassette with the gene of interest linearized with a restriction enzyme that cuts at the 3' side of the overlapping adenoviral genome fragment, preferably not containing any pBr322 vector sequences; and a complementing adenoviral genome construct pWE/Ad.AflII-rITR digested with *PacI.*

These two DNA molecules are further purified by phenol/chloroforme and ETOH precipitation. Co-transfection of these plasmids into an adenovirus packaging cell line, preferably a cell line as PER.C6 or derivative thereof, generates recombinant replication deficient adenoviruses by a one-step homologous recombination between the adapter and the complementing construct.

Recombinant adenovirus can be produced following introduction of said plasmids in said cell. It is to be understood that those skilled in the art may use other combinations of adapter and complementing plasmids without departing from the present invention.

A general protocol as outlined below and meant as a non-limiting example of the present invention has been performed to produce several recombinant adenoviruses using various adapter plasmids and the Ad.AflII-rITR fragment. Adenovirus packaging cells (PER.C6) were seeded in ^{~}25 cm² flasks and the next day when they were at ^{~}80% confluency, were transfected with a mixture of DNA and lipofectamine agent (Life Techn.) as described by the manufacturer. Routinely, 40 µl lipofectamine, 4 µg adapter plasmid and 4 µg of the complementing adenovirus genome fragment AflII-rITR were used. Under these conditions transient transfection efficiencies of ^{~}50% (48 hrs post transfection) were obtained as determined with control transfections using a pAd/CMV-LacZ adapter. Two days later, cells were passaged to ^{~}80 cm² flasks and further cultured. Approximately five later a cytopathic effect (CPE) was seen, indicating that functional adenovirus has formed. Cells and medium are harvested upon full CPE and recombinant virus is released by freeze-thawing. An extra amplification step in a 80 cm² flask was routinely performed to increase the yield since at the initial stage the titers was found to be variable despite the occurrence of full CPE. After amplification, viruses was harvested and plaque purified on PER.C6 cells. Individual plaques were tested for viruses with active transgenes.

The recombinant adenovirus vectors were aliquoted to doses of 1x10¹⁰ iu (equal to approximately 10 x10¹⁰ viral particles) and stored below -20°C until use. Samples were thawed and kept at 4 °C until use.

### Surgical and Perfusion Techniques

Surgical procedures were performed under Hypnorm anaesthesia. (Janssen Pharmaceutica, Tilburg, The Netherlands) For isolated limb perfusion (ILP) a modification of the perfusion technique originally described by Brenckhuijsen was used. After an incision parallel to the inguinal ligament the femoral and vein were approached and cannulated with silastic tubing (0.30 mm ID, 0.64 mm OD; 0.64 mm, 1.19 OD, respectively, Degania Silicone, Degania Bet, Israel). Collaterals were temporarily occluded by the application of a tourniquet around the groin, which was fixed to the inguinal ligament. An oxygenation reservoir and a roller pump (Masterflex), were included in the vascularly isolated circuit, which was, initially, perfused with haemaccel (Behring Pharma, Amsterdam, The Netherlands) for 3 minutes at a flow speed of 2 ml/min to wash out the blood. After the first wash out step, recirculation was performed with recombinant adenoviruses (50 µl-1 ml) dissolved in 2.5-3.5 ml Haemaccel at the same flow rate for a time period of 15 minutes. Followed by a second perfusion step of 5 minutes to wash out the non-bound virus with Haemaccel. During the perfusion and recirculation steps the rat hind leg was kept at a constant temperature of 37-39°, a warm water mattress was applied around the leg. After the second wash-out step, the vascularly isolated circuit was discontinued and, after cannule removal, the femoral vessels were ligated. Previous experiments have shown that the collateral circulation via the internal iliac artery to the leg is so extensive that ligation of the femoral vessels can be performed without detrimental effects.

### Revascularization studies (NB: pre-treatment with haemacell to remove IGG)

Revascularisation was determined by measuring the blood flow in the legs by using a Laser Doppler apparatus.

Rats were used as an animal model, since rats have shown to be suitable for adenoviral vector testing. Rats were anaesthetized by using hypnorm. The blood flow in both hind limbs (the treated right and non-treated left leg) was measured by using a Laser Doppler apparatus according to the standard manufacturers protocol.

Directly after ILP treatment the blood flow in the hind legs was determined again by using the Laser Doppler apparatus.

Until day 30 after treatment every 3 or 4 days the blood flow in the hind limbs was measured by using the Laser Doppler apparatus.

The method of isolated limb perfusion is used to deliver the recombinant adenoviral vectors since it has been shown in previous experiments to be able to deliver adenoviruses to the vasculature of the leg (see also figure 5). Furthermore ILP is a good model to study revascularisation since the artery and vein used for virus delivery are disconnected from the blood circulation causing severe ischemia in the manipulated limb.

### CITED LITERATURE

1. Nakane M, Schmidt HH, Pollock JS, Forstermann U, Murad F FEBS Lett 1993 Jan 25;316(2):175-80 Cloned human brain nitric oxide synthase is highly expressed in skeletal muscle.
2. Janssens SP, Shimouchi A, Quertermous T, Bloch DB, Bloch KD J Biol Chem 1992 Jul 25;267(21):14519-22 Cloning and expression of a cDNA encoding human endothelium-derived relaxing factor/nitric oxide synthase.
3. Geller DA, Lowenstein CJ, Shapiro RA, Nussler AK, Di Silvio M, Wang SC, Nakayama DK, Simmons RL, Snyder SH, Billiar TR Proc Natl Acad Sci U S A 1993 Apr 15;90(8):3491-5 Molecular cloning and expression of inducible nitric oxide synthase from human hepatocytes.
4. Hobbs AJ, Higgs A, Moncada S Annu Rev Pharmacol Toxicol 1999;39:191-220 Inhibition of nitric oxide synthase as a potential therapeutic target.
5. Kay, R., Takei, F., and Humphries, R.K. (1990). Expression cloning of a cDNA encoding M1/69. J. *Immunol.* 145, 1952-1959.
6. Levrero, M., Barban, V., Manteca, S., Ballay, A., Balsamo, C., Avantaggiata, M.L., Natoli, G., Skellekens, H., Tiollais, P., and Perricaudet, M. (1991). Defective and non-defective adenovirus vectors for expression foreign genes in vitro and in vivo. Gene 101, 195-202.

## Claims

1. Use of a nucleic acid delivery vehicle comprising a nucleic acid encoding NOS activity for the manufacture of a pharmaceutical composition for essentially isolated tissue perfusion treatment to enhance and/or induce angiogenesis.

2. Use according to claim 1, wherein the systemic treatment includes isolated limb perfusion.

3. Use according to claim 1 or 2, wherein said nucleic acid delivery vehicle comprises a virus-like particle.

4. Use according to claim 3, wherein the virus-like particle is an adenovirus particle, an adeno-associated virus particle or a retrovirus particle.

5. Means for enhancing and/or inducing angiogenesis comprising a pharmaceutical composition comprising NOS activity.

6. Means according to claim 5, wherein the NOS activity is provided by a recombinant adenoviral vector encoding said activity.

7. Means according to claim 5 or 6, wherein the pharmaceutical composition is a perfusion fluid.

8. Means according to claim 7, wherein the perfusion fluid comprises a virus-like particle comprising a recombinant adenoviral vector.

9. Means according to claim 8, wherein the virus-like particle is present in an amount of from about 10⁶ to about 5.10⁹ iu.

10. A pharmaceutical composition for enhancing and/or inducing angiogenesis comprising NOS activity provided by a nucleic acid delivery vehicle comprising nucleic acid encoding such activity, wherein said pharmaceutical composition is a perfusion fluid.

11. A pharmaceutical composition according to claim 10, wherein the nucleic acid delivery vehicle comprises a virus-like particle.

12. A pharmaceutical composition according to claim 10 or 11, wherein the virus-like particle is present in an amount of from about 10⁶ to about 5.10⁹ iu.

13. A pharmaceutical composition according to claims lo-12, wherein the virus-like particle is an adenovirus particle, an adeno-associated virus particle and/or a retroviral particle.

14. A kit of parts for treatment to enhance and/or induce angiogenesis comprising a pharmaceutical composition according to claims 10-13, means for isolating certain tissues, and means for perfunding said isolated tissues.
